# EUROPEAN PATENT SPECIFICATION

(11) **EP 3 115 071 B1**
(45) Date of publication and mention of the grant of the patent: **27.03.2019**
(21) Application number: 16275087.1
(22) Date of filing: 23.06.2016
(51) Int. Cl.: A61F 2/848, A61M 1/10

(54) **ENDOVASCULAR COMPLIANCE ASSEMBLY**
ANORDNUNG MIT ENDOVASKULÄRER COMPLIANCE
ENSEMBLE DE CONFORMITÉ ENDOVASCULAIRE

(30) Priority: 06.07.2015 US 201514791712
(43) Date of publication of application: 11.01.2017
(73) Proprietor: Cook Medical Technologies LLC, Bloomington, IN 47404 (US)
(72) Inventor: BABBS, Charles F., West Lafayette, IN 47906 (US); CHARLEBOIS, Steven, West Lafayette, IN 47906 (US); HASELBY, Kenneth Aaron, Battle Ground, IN 47920 (US); KRATZBERG, Jarin, Lafayette, IN 47909 (US); METCALF, Justin, West Lafayette, IN 47906 (US); SISKEN, Richard B., West Lafayette, IN 47906 (US)
(74) Representative: Williams Powell

(56) References cited:
- NL-A- 8 100 100
- US-A1- 2009 131 741

## Description

This invention relates generally to medical devices and particularly to an endovascular assembly for improving vascular compliance.

When a vessel loses compliance, it loses elasticity and typically becomes stiffer. Vessels, such as the aorta, can lose compliance due to age, congestive heart failure, atherosclerosis, etc. As the aorta stiffens and loses compliance, the heart struggles to pump blood and must work harder to eject the same volume of blood from the left ventricle into the aorta with each heartbeat. If the heart is incapable of working harder because of underlying diseases, then less blood will be ejected into the aorta with each heartbeat.

NL 8100100 discloses an aorta-implanted balloon optimizing cardiac action of a patient. The balloon is connected to a vessel containing approximately constant gas pressure.

The present invention seeks to provide an improved endovascular assembly.

According to an aspect of the present invention, there is provided an endovascular assembly for improving vascular compliance as specified in claim 1.

Having at least two planar surfaces has the advantage of lessening the probability of blood clots dislodging from the surface of the first container.

The endovascular assembly may further comprise at least one attachment member connectable to the first container, when expanded in a vessel, the attachment member being fixedly positioned therein.

The second container may have a fixed volume or it may have a variable volume.

In an embodiment, the first container comprises an expandable balloon.

The at least one attachment member may be a self-expanding stent.

In an embodiment the second container comprises an outer chamber and an inner inflatable chamber contained within the outer chamber, and wherein the inner chamber is fluidly connectable to the distal end of the first container.

The outer chamber of the second container may comprise a port accessible external to a patient.

The endovascular assembly may further comprise a second attachment member connectable to the first container.

The attachment member may be integrated in a wall of the first container.

In an embodiment, the endovascular assembly for improving vascular compliance comprises: a first container being expandable and endovascular, when expanded in a vessel, the first container having a volume changing in response to blood flowing thereby; the first container having an outer shape with at least two planar surfaces; at least one self-expanding stent connectable to the first container, when expanded in a vessel, the attachment member being fixedly positioned therein; a second container being implantable and fluidly connectable to the first container; wherein when connected, the first container and the second container form a closed fluid system, whereby a change in volume of the first container due to blood flowing thereby is accompanied by a change in pressure of the closed fluid system.

The attachment member may be integrated into at least one planar surface of the first container.

In an embodiment, the endovascular assembly for improving vascular compliance comprises: an expandable balloon, the balloon being endovascular, when expanded in a vessel, the balloon has a variable volume changing in response to blood flowing thereby; the balloon having an outer shape with at least two planar surfaces; a first self-expanding stent connectable to the expandable balloon and a second self-expanding stent connectable to the expandable balloon, when the stents are expanded in a vessel, the stents are fixedly positioned therein; a container comprising an outer chamber and an inner inflatable chamber contained within the outer chamber, the inner chamber being fluidly connectable to the expandable balloon, wherein when connected, the expandable balloon and the inner chamber form a closed fluid system, whereby a change in volume of the balloon due to blood flowing thereby is accompanied by a change in pressure of the closed fluid system.

In one preferred and illustrative embodiment, an endovascular assembly is positionable in the descending aorta of a patient advantageously to improve the compliance of the vessel. As a result, the blood pressure required to pump blood through the vascular system is lowered and the work the heart performs to pump the same volume of blood through the vascular system is reduced. If the work of the heart remains constant, then a greater volume of blood will be pumped.

To accomplish this, the endovascular assembly in the preferred and illustrative embodiment includes a first container, such as an expandable balloon, that is positionable within a vessel such as, for example, the descending aorta. The first container is fixedly positionable within the vessel with at least one attachment member. The first container has a shape and a volume at least one thereof that is variable and changes as blood in the vessel flows thereby. The first container is fluidly connectable to a second container, which is preferably located outside of the vessel, to form a closed fluid system. The connecting fluid may be a gas such as air or carbon dioxide.

When the first container and second container are connected and inflated with a volume of fluid such as a gas or a liquid, the second container acts as a reservoir for fluid to flow in and out of the first container. The first container decreases in volume as blood is expelled from the left ventricle of the heart and flows over the first container. When the balloon decreases in volume, fluid in the first container flows into the second container. In a preferred embodiment of the present invention, the second container has a fixed volume, and a rigid wall. In another embodiment of the present invention the second container has an elastic wall and a variable volume.

Advantageously, the second container is implantable and preferably placed in the abdomen or subcutaneous tissue of the leg, requiring no external bodily connections to a power source or pump. By not relying on external connections, the endovascular assembly functions as a passive pump is and preferably used for more long-term care, particularly in ambulatory patients. Moreover, the second container is preferably divided into two compartments, an inner inflatable chamber that is directly connected to the first container, and an outer chamber. The outer chamber preferably includes a port which advantageously allows for a physician to make adjustments, such as fluid volume, to the endovascular assembly after initial implantation. In this embodiment, the fluid in the inner chamber and first container would preferably be carbon dioxide or another gas safe for use in the bloodstream, while the fluid in the outer chamber could be the same or another fluid.

The attachment member is preferably a self-expanding stent located externally to the first container. Preferably, there are two self-expanding stents connected to the proximal and the distal ends of the first container. In another embodiment, the attachment member is preferably a self-expanding balloon, located internally in and/or integrally with the first container.

Embodiments of the present invention are described below, by way of example only, with reference to the accompanying drawings, in which:
FIG. 1 depicts a side plan view of an illustrative embodiment of an endovascular assembly within the descending aorta of a patient, depicting a first container within the aorta connected to a second container located in the abdomen;
FIG. 2 depicts a side plan view of an intra-aortic balloon and attachment members of the endovascular assembly of FIG. 1;
FIGS. 3A-3C depict cross-sectional views of the intra-aortic balloon of FIG. 2 within a vessel;
FIG. 4 depicts an enlarged side plan view of the endovascular assembly of FIG. 1 including the intra-aortic balloon and an extravascular container;
FIGS. 5A-5B depict side plan views of the endovascular assembly including the intra-aortic balloon and the extravascular container with an outer chamber and an inner inflatable chamber inflated with a fluid such as a gas or a liquid; and
FIGS. 6A-6C depict a side plan view of the endovascular assembly with an external attachment member(s), and two cross sectional views of the intra-aortic balloon with alternative stent placements.

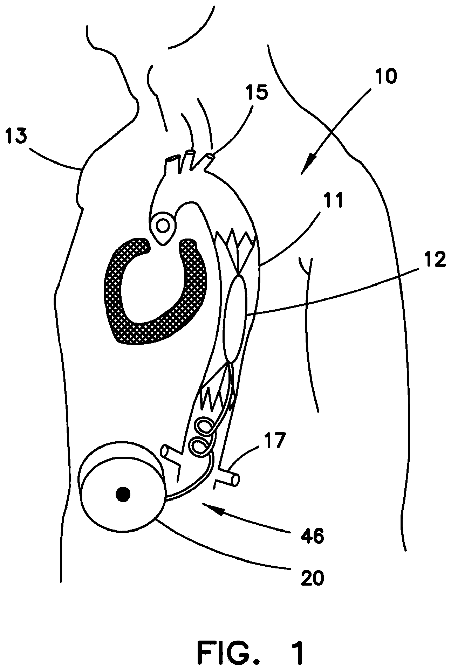

Now looking at the drawings and in particular FIG. 1, an illustrative embodiment of an endovascular assembly 10 is depicted positioned in the descending aorta 11 of a patient 13. The endovascular assembly 10 includes a first container 12 that is expandable and is placed in the aorta. A second container 20 located outside of the vasculature is placed subdermally and connected to the first container 12 to form a closed fluid system 46. The first and second containers are inflated with a volume of fluid such as a gas or a liquid. The second container 20 serves as a reservoir for fluid to flow into and out of the first container 12. As the first container 12 decreases in volume and collapses under the increasing pressure of blood being ejected from the left ventricle, a volume of fluid in the first container 12 flows into the second container 20. By collapsing under increasing pressure of the passing blood, the first container 12 adds compliance to the aorta which allows the heart to eject the same amount of blood at a reduced blood pressure or a greater volume of blood at a constant pressure. The movement of fluid between the first container 12 and the second container 20 is ideally passive as according to Boyle's law, **P₁V₁=P₂V₂** (P=pressure and V=volume) In a closed system a change in fluid pressure or volume in one chamber will result in the equivalent change in fluid pressure or volume in the connected chamber. Additionally the fluid volume in the first container 12 will change with changes in pressure at a given temperature to maintain equilibrium, according to the ideal gas law **PV=nRT** (P=pressure, V=volume, n=number of moles of fluid, R=ideal gas constant, T=temperature).

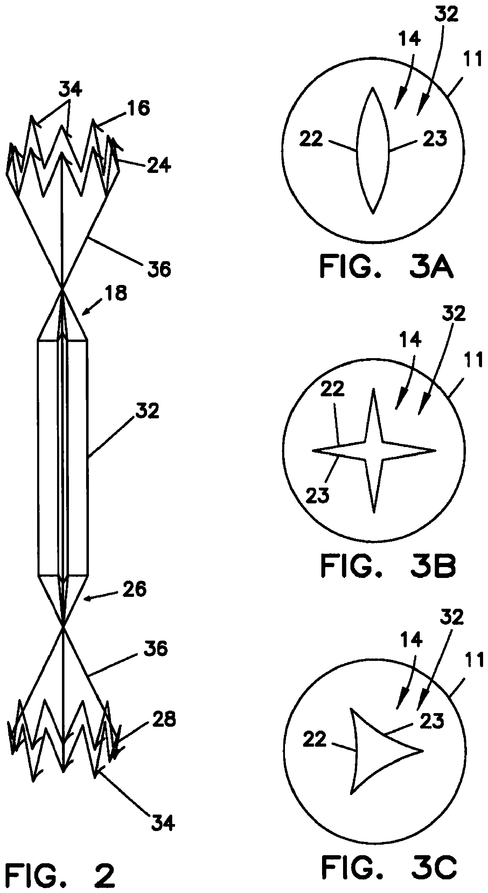

FIG. 2 depicts a side plan view of the intra-aortic balloon of endovascular assembly 10 of FIG. 1. The intra-aortic balloon 32 has a proximal end 18 and distal end 26 and a diameter and cross sectional shape that allows blood to flow past it in the aorta 11.

FIGS. 3A-C depict the balloon 32 of FIG. 2 having an outer shape 14 with at least two planar surfaces 22 and 23. It is also desirable to have a balloon with multiple planar surfaces 22 and 23, such as a star shaped balloon 32, depicted in FIGS. 2 and 3B, or a triangular shaped balloon 32, depicted in FIG. 3C. It is advantageous to have a balloon shaped with at least two generally planar surfaces 22 and 23, although some curvature is permitted. Balloons with generally planar surfaces, as opposed to cylindrical or spherical balloons, commonly prevent clots from dislodging from the surface of the balloon. Additionally, it is desirable for both the proximal and distal portions of the balloon to be tapered to lessen resistance to blood flow.

The intra-aortic balloon 32 is preferably made of polymer materials such as a polyamide-polyimide blend, Thoralon®, silicone, or nylon; however, it should be understood that a variety of other commercially available materials can be used.

Referring now to FIG. 2, it can be seen that the intra-aortic balloon 32 is connected to at least one attachment member 16. The attachment member is preferably a self-expanding stent 24. The stent 24 serves to anchor and centre the balloon 32 within the aorta. The stent 24 can preferably have a Z-stent configuration or be a cannula cut stent (U.S. Patent 7,905,915 and U.S. Patent 7,172,623). The stent 24 is preferably made of nitinol, but can be made out of stainless steel, or any other suitable commercially available material for endovascular stents. Further, it may be advantageous for the stent 24 to contain barbs 34 that engage with the wall of the aorta to enhance attachment to the aortic wall.

There is at least one self-expanding stent 24 connected to the proximal end 18 of the intra-aortic balloon 32, or preferably two self-expanding stents with the second stent 28 connected to the distal end 26 of the balloon 32. The balloon 32 is connected to the self-expanding stents by tethers 36. The tethers 36 may be non-resorbable, commercially available sutures of any suitable size made out of a variety of biocompatible materials. However, it may be advantageous to use chromic sutures that can be broken with a balloon. This would allow for the endovascular assembly 10 to be removed if it should become necessary sometime after implantation.

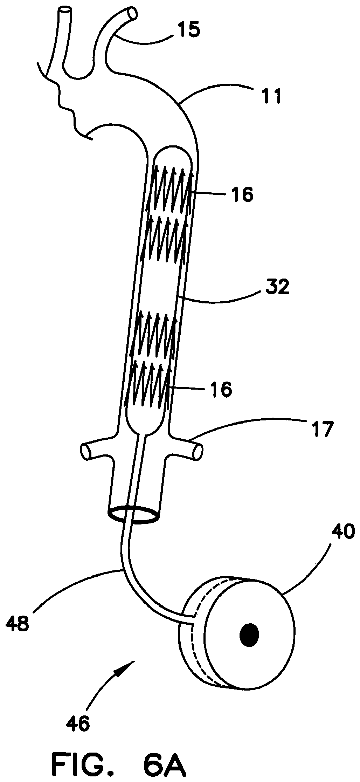

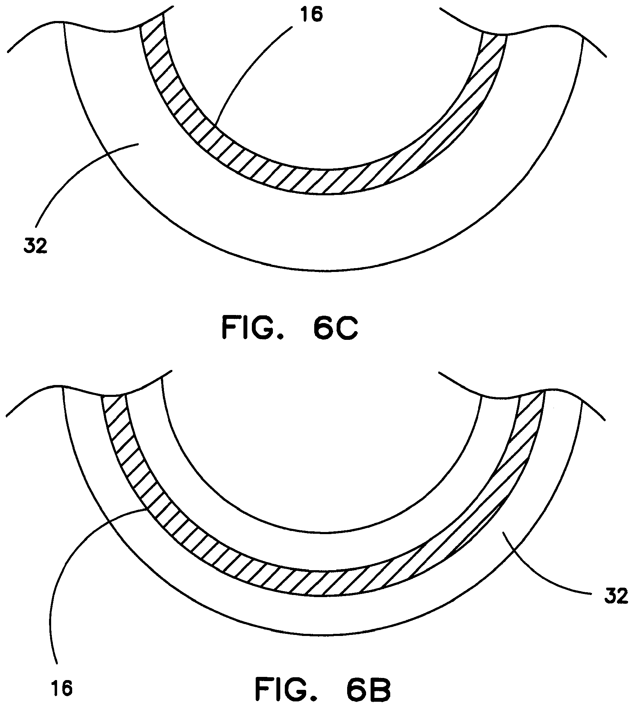

In another embodiment, depicted in Fig. 6A-C, the connectable attachment member 16 may be integrated into the design of the intra-aortic balloon 32 itself. Where connectable refers to circumferentially, longitudinally, internally, or externally connectable to the intra-aortic balloon 32. The attachment member 16 is a self-expanding stent located internally of (FIG. 6C) or as in FIG. 6B in the wall of the intra-aortic balloon 32. An integrated stent and balloon construct would not require tethers and would allow the balloon material to flex as needed while the integrated stent maintains a grip on the wall of the aorta. The stent is preferably self-expanding and can be flat or rounded where it contacts the wall of the aorta. The integrated stent is preferably made of nitinol, or any other suitable commercially available stent material, to allow the integrated stent and balloon construct to be folded like a conventional balloon for delivery. In another embodiment, the endovascular assembly 10 comprises no attachment members 16.

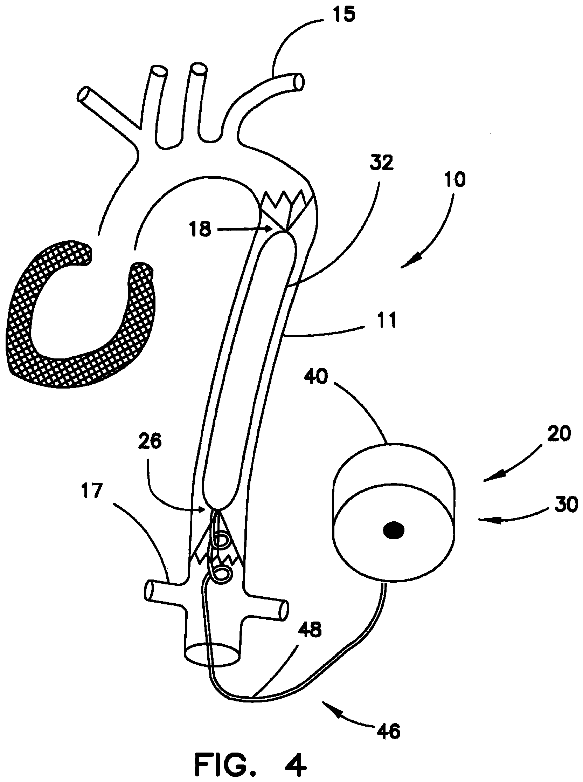

FIG. 4 depicts an enlarged side plan view of the endovascular assembly 10 of FIG. 1 including the intra-aortic balloon 32 and an extravascular container 30. The intra-aortic balloon 32 is endovascularly positioned in the descending aorta 11 and is placed so that its proximal end 18 lies just distal to the subclavian arteries 15, and its distal end 26 lies just above the renal arteries 17. The length of the balloon 32 for a typical adult would be approximately 20 centimeters in length, but may be adjusted to suit different patient vascular anatomies. Positioned in such a manner, clots that may form and dislodge from the balloon would avoid the brain. Furthermore, concerns regarding blockage of renal or mesenteric arteries are reduced.

It can be seen that the intra-aortic balloon 32, as depicted in FIG. 4, is fluidly connected to the second container 20, or extravascular container 30, by a hollow flexible tube 48. The connection between the intra-aortic balloon 32 and extravascular container 30 forms a closed fluid system 46 such that fluid, such as a gas or a liquid, within the balloon 32 can flow into the extravascular container 30, or fluid within the extravascular container 30 can flow into the intra-aortic balloon 32, but the fluid will not enter the vascular system. The diameter of the tube or catheter 48 connecting the extravascular container 30 will typically be at least 2mm to avoid introducing significant resistance to fluid flow between the balloon 32 and the extravascular container 30. The catheter 48 may also have a curved or spiraled portion to avoid placing the aorta and iliac arteries under undue longitudinal stress.

The intra-aortic balloon 32 is inflated with a fluid such as a gas or a liquid. The balloon 32 is preferably inflated with carbon dioxide or any other suitable fluid that is impermeable to the material of the balloon. Carbon dioxide is preferred for safety, as it would rapidly dissolve into the bloodstream in the event of system rupture.

The extravascular container 30 is preferably implanted subdermally, preferably below the surface of the abdomen. The catheter 48 can be tunneled from the location that it exits the aorta to the extravascular container 30. In another embodiment of the present invention, the extravascular container 30 can be implanted in the patient's 13 shoulder or chest area, allowing the catheter 48 to travel through the subclavian artery and the balloon 32 to hang freely generally below the container 30, such as in the descending aorta 11. The extravascular container 30 and or outer chamber 40 can be of either elastic or rigid material in this embodiment. Where the extravascular container 30 and or chamber 40 are elastic, their volumes will be variable.

The outer chamber 40 of the extravascular container 30 is preferably made out of an elastic or rigid material that will not burst or rupture upon a forceful impact that could result, for example, by a patient falling. Thus, the outer chamber 40 can be made out of polyether ether ketone (PEEK), high density polyethylene (HDPE), a polyamide-polyimide blend, or any other similar commercially available material suitable for implantation within a human patient.

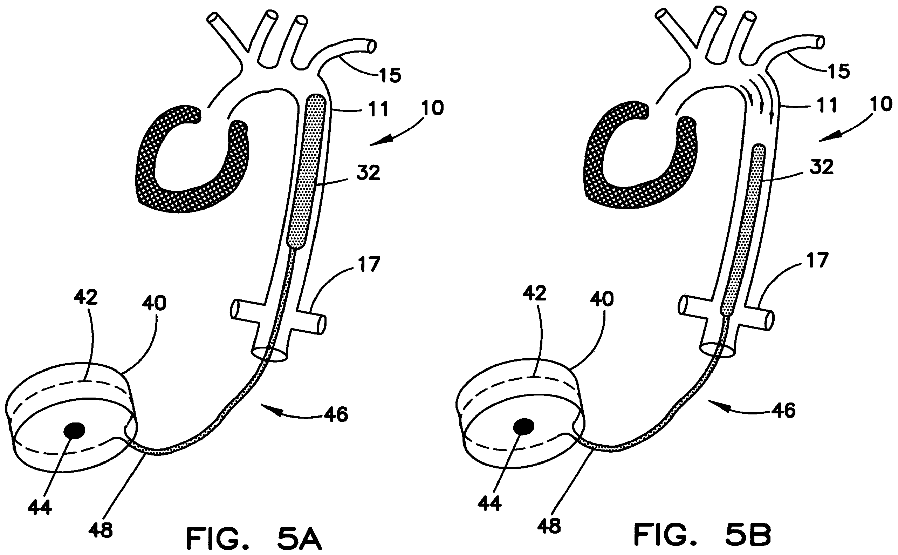

FIGS. 5A-5B depict side plan views of an embodiment of endovascular assembly including the intra-aortic balloon and the extravascular container with an outer chamber and inner inflatable chamber inflated with a gas or a liquid. It can be seen that the extravascular container 30 is preferably divided into two compartments. The first compartment, or inner inflatable chamber 42, is directly connected to the intra-aortic balloon 32 and resides inside the larger second compartment, or outer chamber 40. The inner inflatable chamber 42 can be made of the same material as the intra-aortic balloon 32. The inner inflatable chamber 42 isolates the fluid in the outer chamber from the fluid in the catheter 48 and intra-aortic balloon 32. This acts as a safety feature in the event of a balloon rupture. It also allows for different fluids to be utilized in the balloon 32 and inner inflatable chamber 42 than in the outer chamber 40. For example, the balloon 32 and inner inflatable chamber 42 can be inflated with carbon dioxide while the outer chamber 40 can be inflated with another fluid. This may be advantageous as it may be convenient for adjusting the pressure of the endovascular assembly to meet patient needs.

In this regard, it would be desirable to include a port 44 on the outer chamber of the second container that is accessible external to the patient. This would be advantageous to allow for adjustment of the pressure in the closed fluid system 46. The port can be a septum or an infusion port 44 similar to the Vital-Port Titanium Power-Injectable Vascular Access System manufactured by COOK Medical Technologies, Bloomington, IN. As the outer chamber is implanted subdermally in the abdomen or thigh, a physician can easily access the port in order to increase or decrease the pressure of gas or liquid in the closed fluid system. This could be done in an outpatient procedure.

Another possible feature of the extravascular container 30 involves one or more pressure sensors (not shown) and sufficient electronics to transmit the pressures measured through the patient to an external reader. The electronics may be self-powered with batteries or energy harvesting technologies (e.g. piezoelectric or photovoltaic systems) or powered by radio frequency or other electromagnetic energy from the external reader or other external source.

When the intra-aortic balloon 32 and the inner inflatable chamber 42 of the extravascular container 30 are connected as depicted in FIGS. 5A and 5B, a closed fluid system 46 is formed. The balloon 32 is inflated with a volume of gas or liquid in the range of about 50 mL to about 130 mL. The outer chamber 40 would have a volume capacity in the range of about 80 mL to about 120 mL and the inner inflatable chamber would have a volume in the range of about 60mL and about 95mL, or approximately 80 to 90% of the total capacity of the outer chamber. Initially, the intra-aortic balloon 32 and inner inflatable chamber 42 can be inflated with carbon dioxide. Sometime after deployment, it may desirable to adjust the volume of fluid in the endovascular assembly 10. To achieve this, the outer chamber 40 can be inflated with for example, air, or any other suitable fluid, via the infusion port 44 provided on the outer chamber 40. It may be advantageous to inflate the outer chamber 40 with air as this could be done in a simple outpatient procedure.

When the intra-aortic balloon 32 and the inner inflatable chamber 42 of the extravascular container are connected and inflated in the above described manner and as depicted in FIG. 5A, the balloon 32 acts as passive pump requiring no open wounds to connect the balloon to an external pump or power supply. The extravascular container 30 serves as a volume compensating reservoir for the intra-aortic balloon 32. As the balloon collapses, depicted in FIG. 5B under the increasing pressure of blood being ejected from the heart, a volume of fluid in the balloon 32 flows into the inner inflatable chamber 42 of the extravascular container 30. By collapsing under increasing pressure, the balloon 32 adds compliance to the aorta which allows the heart to eject the same amount of blood with less pressure or to eject a greater amount of blood with the same pressure.

## Claims

1. An endovascular assembly (10) for improving vascular compliance including:
a first container (12) being expandable and endovascular, when expanded in a vessel, the first container having a volume changing in response to blood flowing thereby and an outer shape with at least two planar surfaces (22, 23); and
a second container (20) being implantable and fluidly connectable to the first container;
wherein when connected, the first container and the second container form a closed fluid system, whereby a change in volume of the first container due to blood flowing thereby is accompanied by a change in pressure of the closed fluid system.

2. An endovascular assembly (10) as claimed in claim 1 including at least one attachment member (16) connectable to the first container (12), when expanded in a vessel, the attachment member being fixedly positioned therein.

3. An endovascular assembly (10) as claimed in claim 2, wherein the at least one attachment member (16) is a self-expanding stent (24).

4. An endovascular assembly (10) as claimed in claim 2 or 3, wherein the attachment member (16) is integrated in a wall of the first container (12).

5. An endovascular assembly (10) as claimed in claim 2, 3 or 4, including a second attachment member (16) connectable to the first container (12).

6. An endovascular assembly (10) as claimed in claim 5, wherein the second attachment member (16) is a self-expanding stent (28).

7. An endovascular assembly (10) as claimed in any of claims 2 to 6, wherein the attachment member (16) is integrated into at least one planar surface of the first container.

8. An endovascular assembly (10) as claimed in any preceding claim, wherein the second container (20) has a fixed volume.

9. An endovascular assembly (10) as claimed in any of claims 1 to 7, wherein the second container (20) has a variable volume.

10. An endovascular assembly (10) as claimed in any preceding claim, wherein the first container (12) comprises an expandable balloon (32).

11. An endovascular assembly (10) as claimed in any preceding claim, wherein the second container (20) includes an outer chamber (40) and an inner inflatable chamber (42) contained within the outer chamber, and wherein the inner chamber is fluidly connectable to the distal end of the first container (12).

12. An endovascular assembly (10) as claimed in claim 11, wherein the outer chamber (40) of the second container (20) comprises a port (44) accessible externally of a patient (13).

13. An endovascular assembly (10) as claimed in any preceding claim:
wherein the first container (12) comprises an expandable balloon (32), the balloon being endovascular, when expanded in a vessel, the balloon having a variable volume changing in response to blood flowing thereby;
wherein the balloon has an outer shape with at least two planar surfaces (22, 23);
wherein the assembly includes a first self-expanding stent (24) connectable to the expandable balloon and a second self-expanding stent (28) connectable to the expandable balloon, when the stents are expanded in a vessel, the stents being fixedly positioned therein;
wherein the second container (20) includes an outer chamber (40) and an inner inflatable chamber (42) contained within the outer chamber, the inner chamber being fluidly connectable to the expandable balloon.

## Patentansprüche

1. Endovaskuläre Anordnung (10) zur Verbesserung der vaskulären Compliance, einschließend:
einen ersten Behälter (12), der expandierbar und endovaskulär ist, wenn er in einem Gefäß expandiert wird, wobei der erste Behälter ein Volumen, das sich in Reaktion auf hindurch fließendes Blut ändert, und eine äußere Form mit mindestens zwei planaren Oberflächen (22, 23) aufweist; und
einen zweiten Behälter (20), der implantierbar und fließtechnisch mit dem ersten Behälter verbindbar ist;
wobei der erste Behälter und der zweite Behälter, wenn sie verbunden sind, ein geschlossenes Fließsystem bilden, wodurch eine Änderung des Volumens des ersten Behälters infolge des hindurch fließenden Blutes von einer Druckänderung des geschlossenen Fließsystems begleitet wird.

2. Endovaskuläre Anordnung (10) nach Anspruch 1, die mindestens ein Befestigungselement (16) einschließt, das mit dem ersten Behälter (12) verbindbar ist, wobei das Befestigungselement bei Expansion in einem Gefäß darin fest positioniert ist.

3. Endovaskuläre Anordnung (10) nach Anspruch 2, wobei das mindestens eine Befestigungselement (16) ein selbstexpandierender Stent (24) ist.

4. Endovaskuläre Anordnung (10) nach Anspruch 2 oder 3, wobei das Befestigungselement (16) in eine Wand des ersten Behälters (12) integriert ist.

5. Endovaskuläre Anordnung (10) nach Anspruch 2, 3 oder 4, die ein zweites Befestigungselement (16) einschließt, das mit dem ersten Behälter (12) verbindbar ist.

6. Endovaskuläre Anordnung (10) nach Anspruch 5, wobei das zweite Befestigungselement (16) ein selbstexpandierender Stent (28) ist.

7. Endovaskuläre Anordnung (10) nach einem der Ansprüche 2 bis 6, wobei das Befestigungselement (16) in mindestens eine planare Oberfläche des ersten Behälters integriert ist.

8. Endovaskuläre Anordnung (10) nach einem der vorhergehenden Ansprüche, wobei der zweite Behälter (20) ein festes Volumen aufweist.

9. Endovaskuläre Anordnung (10) nach einem der Ansprüche 1 bis 7, wobei der zweite Behälter (20) ein variables Volumen aufweist.

10. Endovaskuläre Anordnung (10) nach einem der vorhergehenden Ansprüche, wobei der erste Behälter (12) einen expandierbaren Ballon (32) aufweist.

11. Endovaskuläre Anordnung (10) nach einem der vorhergehenden Ansprüche, wobei der zweite Behälter (20) eine äußere Kammer (40) und eine innere aufpumpbare Kammer (42) einschließt, die in der äußeren Kammer enthalten ist, und wobei die innere Kammer fließtechnisch mit dem distalen Ende des ersten Behälters (12) verbindbar ist.

12. Endovaskuläre Anordnung (10) nach Anspruch 11, wobei die äußere Kammer (40) des zweiten Behälters (20) einen Anschluss (44) umfasst, der außerhalb eines Patienten (13) zugänglich ist.

13. Endovaskuläre Anordnung (10) nach einem der vorhergehenden Ansprüche,
wobei der erste Behälter (12) einen expandierbaren Ballon (32) umfasst, wobei der Ballon endovaskulär ist, wenn er in einem Gefäß expandiert wird, wobei der Ballon ein variables Volumen aufweist, das sich in Reaktion auf hindurch fließendes Blut ändert;
wobei der Ballon eine äußere Form mit mindestens zwei planaren Oberflächen (22, 23) aufweist;
wobei die Anordnung einen ersten selbstexpandierbaren Stent (24), der mit dem expandierbaren Ballon verbindbar ist, und einen zweiten selbstexpandierbaren Stent (28) einschließt, der mit dem expandierbaren Ballon verbindbar ist, wobei, wenn die Stents in einem Gefäß expandiert werden, die Stents darin fest positioniert sind;
wobei der zweite Behälter (20) eine äußere Kammer (40) und eine innere aufpumpbare Kammer (42) einschließt, die innerhalb der äußeren Kammer enthalten ist, wobei die innere Kammer fließtechnisch mit dem expandierbaren Ballon verbindbar ist.

## Revendications

1. Ensemble endovasculaire (10) permettant d'améliorer une conformité vasculaire comprenant :
un premier contenant (12) pouvant être déployé et endovasculaire, lorsqu'il est déployé dans un vaisseau, le premier contenant ayant un volume changeant en réponse à du sang circulant par celui-ci et une forme externe ayant au moins deux surfaces planes (22, 23) ; et
un second contenant (20) pouvant être implanté et relié fluidiquement au premier contenant ;
dans lequel, lorsqu'ils sont reliés, le premier contenant et le second contenant forment un système fluide fermé, moyennant quoi un changement de volume du premier contenant dû au sang circulant par celui-ci est accompagné d'un changement de pression du système de fluide fermé.

2. Ensemble endovasculaire (10) selon la revendication 1 comprenant au moins un élément de fixation (16) pouvant être relié au premier contenant (12), lorsqu'il est déployé dans un vaisseau, l'élément de fixation étant positionné à demeure en son sein.

3. Ensemble endovasculaire (10) selon la revendication 2, dans lequel l'au moins un élément de fixation (16) est un stent autodéployable (24).

4. Ensemble endovasculaire (10) selon la revendication 2 ou 3, dans lequel l'élément de fixation (16) est intégré dans une paroi du premier contenant (12).

5. Ensemble endovasculaire (10) selon la revendication 2, 3 ou 4, comprenant un second élément de fixation (16) pouvant être relié au premier contenant (12).

6. Ensemble endovasculaire (10) selon la revendication 5, dans lequel le second élément de fixation (16) est un stent autodéployable (28).

7. Ensemble endovasculaire (10) selon l'une quelconque des revendications 2 à 6, dans lequel l'élément de fixation (16) est intégré dans au moins une surface plane du premier contenant.

8. Ensemble endovasculaire (10) selon l'une quelconque des revendications précédentes, dans lequel le second contenant (20) a un volume fixe.

9. Ensemble endovasculaire (10) selon l'une quelconque des revendications 1 à 7, dans lequel le second contenant (20) a un volume variable.

10. Ensemble endovasculaire (10) selon l'une quelconque des revendications précédentes, dans lequel le premier contenant (12) comprend un ballonnet expansible (32).

11. Ensemble endovasculaire (10) selon l'une quelconque des revendications précédentes, dans lequel le second contenant (20) comprend une chambre externe (40) et une chambre gonflable interne (42) contenue dans la chambre externe, et dans lequel la chambre interne peut être reliée fluidiquement à l'extrémité distale du premier contenant (12).

12. Ensemble endovasculaire (10) selon la revendication 11, dans lequel la chambre externe (40) du second contenant (20) comprend un orifice (44) accessible de l'extérieur d'un patient (13).

13. Ensemble endovasculaire (10) selon l'une quelconque des revendications précédentes :
dans lequel le premier contenant (12) comprend un ballonnet expansible (32), le ballonnet étant endovasculaire, lorsqu'il est déployé dans un vaisseau, le ballonnet ayant un volume variable changeant en réponse au sang circulant par celui-ci ;
dans lequel le ballonnet a une forme externe ayant au moins deux surfaces planes (22, 23) ;
dans lequel l'ensemble comprend un premier stent autodéployable (24) pouvant être relié au ballonnet expansible et un second stent autodéployable (28) pouvant être relié au ballonnet expansible, lorsque les stents sont déployés dans un vaisseau, les stents étant positionnés à demeure en son sein ;
dans lequel le second contenant (20) comprend une chambre externe (40) et une chambre gonflable interne (42) contenue dans la chambre externe, la chambre interne pouvant être reliée fluidiquement au ballonnet expansible.
